# EUROPEAN PATENT APPLICATION

(11) **EP 2 832 322 A1**
(43) Date of publication of application: **04.02.2015**
(21) Application number: 13178604.8
(22) Date of filing: 30.07.2013
(51) Int. Cl.: A61F 2/966, A61F 2/95

(54) **Deployment system for a cardiac valve prosthesis**

(71) Applicant: NVT AG, 5630 Muri AG (CH)
(72) Inventor: Stockert, Gunther, 72076 Tübingen (DE); Kawa, Emilia, 72379 Hechingen (DE); Centola, Marcos, Sao Paulo (BR); Drescher, Christian, 99867 Gotha (DE)
(74) Representative: Witte, Weller & Partner Patentanwälte mbB

(57) **Abstract**

The present invention relates to a deployment system (10) for deploying a self expandable prosthesis (22), the deployment system (10) having a proximal (11) and a distal (12) end, and a longitudinal axis (13) extending from the proximal (11) to the distal (12) end. It further comprises a first tube (14) having a distal (21) tip element end, holding the proximal end of the prosthesis (22). The deployment system (10) also comprises a sheath tube which holds and compresses t a distal end (23) of the prosthesis (22) in the loaded state. The deployment system (10) further comprises an actuating mechanism (30) by means of which the axial movement of the tip element (18) releasing the proximal end (24) of the prosthesis (22) and the axial movement of the sheath tube (26) releasing the distal end (23) of the prosthesis (22) are controlled and operated separately from one another, wherein the actuating mechanism (30) comprises a switching element (32) being operable to perform different switching steps (P, N, 1, 2, 3, 4) (Fig. 1A).

## Description

The present invention relates to a deployment system for deploying a self expandable prosthesis, in particular of a cardiac valve prosthesis.

Heart valve replacement is necessary where the native heart valve is damaged, mal- or nonfunctioning. In the heart, cardiac - or "aortic" - valves maintain the unidirectional flow of blood by opening and closing depending on the difference in pressure on each side.

The aortic valve can be affected by a range of diseases and can, therefore, require cardiac valve replacement, which means that a patient's aortic valve is replaced by a different valve. The valve can either become leaky, i.e. regurgitant or insufficient, in which case the aortic valve is incompetent and blood flows passively back to the heart in the wrong direction. Further, the valve can become partially shut, i.e. stenotic, in which case the valve fails to open fully, thereby obstructing blood flow out from the heart. The two conditions frequently co-exist.

There are two basic types of artificial heart valve, mechanical valves and tissue valves. Mechanical heart valves are generally composed entirely of synthetic or nonbiological materials, whereas tissue (or bioprosthetic) heart valves are composed of synthetic and biological materials. Bioprosthetic cardiac valves can either represent xenografts, which are taken from different species than the recipient, or homografts, which are donor valves taken from the same species as the recipient. Generally, the artificial valves comprise expandable stent systems which are introduced into the vessel in a compressed state and which are allowed to expand by removal of compressive structures.

Aortic valve replacement traditionally requires median sternotomy and thus open heart surgery, which is a major impact on the patient to be treated: The chestbone is sawed in half and after opening of the percardium, the patient is placed on a cardiopulmonary bypass machine. Once the patient is on bypass, the patient's diseased aortic valve is removed and a mechanical or tissue valve is put in its place. Besides the physical stress associated with this operation, there is a risk of death or serious complications from open heart surgery, in particular depending on the health and age of the patient.

However, recently, valves are developed that can be implanted using a catheter or deployment systems without open heart surgery, but via vascular access, and the deployment of the prosthesis can either be achieved retrograde, i.e. against normal blood flow, or antegrade, with blood flow.

Amongst the possible vascular access possibilities are the femoral, subclavian, transaortic and transapical access, wherein a transcatheter aortic valve implantation is - generally - abbreviated with TAVI, and wherein the transfemoral access wit TFAVI, and the transapical technique with TA-AVI.

In particular the TA-AVI as a minimal invasive technique has been proven to be a promising access in order to avoid the surgical risks involved with conventional aortic valve replacement, in particular with elderly people. With using transcatheer technique it is feasible to implant an aortic valve prosthesis and avoid sternotomy, cardioplegic arrest and probably most importantly even cardiopulmonary bypass.

However, up to today, only a very few heart valve prostheses are available for transcatheter access in general, and for transapical access in particular, since correct and efficient deployment of the prosthesis remains a difficult task for the surgeon.

International patent application WO 2008/070797 discloses a system and a method for transapical delivery of an annulus anchored self-expanding valve. The system disclosed therein comprises a catheter assembly having an outer sheath, an elongate pusher tube and a central tube. At the distal end of the central tube an atraumatic tip is provided; in the loaded state, the prosthesis is carried adjacent to the tip on the central tube and compressed by the sheath. Upon retraction of the sheath, the distal end of the prosthesis, i.e. the one that is located nearest to the tip is released and the prosthesis is allowed to expand. Subsequently, the sheath gets completely pulled back to fully release the prosthesis, which subsequently, in case of as self-expanding prosthesis, can fully expand, or which can be expanded by using a balloon.

Similarly, WO 2007/098232 discloses a deployment device for self-expanding prostheses, wherein the device comprises a split sheath by means of which the prosthesis can be deployed in several steps.

Despite the many different deployment systems and techniques known in the art, the precise deployment of the prosthesis still remains a crucial step and is difficult to achieve with the systems, devices and methods presently available. The available deployment systems all necessitate the surgeon to work and deploy the prosthesis under time pressure to avoid disruption of normal blood flow.

Against this background it is an object of the invention to provide for an improved deployment system by means of which an obstruction of the blood flow, and, thus, an impairment of the heart's natural function, can be prohibited, whilst simultaneously, the deployment system as such should be easy to handle, and allow for precise placement and eventually of correction of a misplaced prosthesis.

According to the invention, this object is achieved by a deployment system as described in detail in the claims, which according to one aspect of the invention is used in a transapical access for transcatheter aortic valve replacement.

In particular, with the present invention a deployment system for deploying a self expandable prosthesis is provided, the deployment system having a proximal and a distal end, and a longitudinal axis extending from the proximal to the distal end, and further comprising: a first tube comprising a lumen, and comprising a tip element having a proximal and a distal tip element end, the tip element being fixedly connected to the first tube at a distal end of the first tube, wherein the tip element is designed such, that a proximal end of a prosthesis is detachably accomodatable and holdable by and within the tip element, wherein the tip element is slidable distally relative to the prosthesis; the deployment system further comprises a sheath tube having a proximal and a distal sheath tube end, the sheath tube being designed such, that a distal end of the prosthesis is detachably holdable and compressable by and within the distal sheath tube end, wherein the sheath tube is slidable proximally relative to the prosthesis, to release the distal end of the prosthesis; in the deployment system according to the invention, the first tube and the sheath tube are coaxially arranged with respect to one another and are axially moveable with respect to one another, and wherein the tip element and the sheath tube, in a loaded state of the cardiac valve prosthesis, are arranged such, that the proximal tip element end and the distal sheath tube end abut to one another, or that the proximal tip element end is slidable over the distal sheath tube end, to release and expand the prosthesis; the deployment system according to the invention also further comprises an actuating mechanism by means of which the axial movement of the tip element releasing the proximal end of the prosthesis and the axial movement of the sheath tube releasing the distal end of the prosthesis are controllable independently from one another, wherein the actuating mechanism comprises a switching element being operable to perform different switching steps.

In the prior art, and in the present application, the end of a prosthesis which is to be placed nearer to the heart is designated as "proximal end", whereas the end of the prosthesis to be implanted farther away from the heart, is designated as "distal" end. Accordingly, the end of a cardiac valve prosthesis (or "heart valve") comprising the valve, is usually called the "proximal end", whereas the end of the heart valve further free from valve material is called the "distal end".

In contrast, the designation of the ends or end portions of the deployment system as distal and proximal is such that the end closer to the operator is designated as "proximal" proximal and the other end further from the operator is designated "distal". Presently and throughout this application, if the expression "proximal" is used in connection with designating a direction, i.e. the "proximal direction", the direction towards the operator is meant. On the other hand, if the expression "distal" is used to describe a direction, i.e. the "distal direction", the direction leading or pointing away from the operator/surgeon/practitioner is meant. Also, when designating the ends of the parts of the deployment system, the term "proximal end" refers to the end that is nearest to the operator, e.g. the surgeon, and the term "distal end" refers to the end opposite to the proximal end and is nearer to the patient to be treated.

Presently, with the expression "operated and controlled separately," it is meant that the movement of the tip element and the movement of the sheath element can/ are effected separately from one another, while at the same time control elements for the movement of the tip element and the sheath element may be/are linked, e.g. via an internal bushing element. According to a preferred embodiment,

With the deployment system according to the invention, on the one hand, the blood flow during deployment of the prosthesis is guaranteed, since a flaring of the proximal end prior to the correct placement is prevented which flaring otherwise would block the blood flow. Also, on the other hand, a deployment of the distal end of the prosthesis prior to the deployment of the proximal end of the prosthesis, which would obstruct blood flow, is successfully prevented. This is achieved by the tip accommodating the proximal end of the prosthesis, and by the fact that the distal end of the prosthesis remains attached and in a compressed state after the proximal end has been released. Further, with the deployment system according to the invention, a very secure system is provided, since a too rapid or premature releasing of one or the other end of the prosthesis is actively prevented by means of the actuating system. Thus, even in highly stressful situations - as heart valve replacement processes always represent - an erroneous or false or too fast handling of the deployment system is practically prevented.

With the deployment system according to the invention and the novel actuating mechanism comprised therein, the axial movement of the tip element releasing the proximal end of the prosthesis and the axial movement of the sheath tube releasing the distal end of the prosthesis are controllable independently from one another. This is due to the actuating mechanism comprising a switching element being operable to perform different switching steps and preventing that the first tube is operated at the same time as the sheath tube, so that an accidental deployment of the prosthesis is effectively prevented.

In a preferred embodiment according to the invention, the switching element, as one switching step, is operable such that the movement of the tip element fixed to the first tube is effected when the movement of the sheath tube is blocked.

Further preferably, the switching element, as a second switching step, is operable such that the movement of the tip element fixed to the first tube is blocked when the movement of the sheath tube is effected.

Also, it is further preferred if the switching element, as a third switching step, is operable such that movement of both, the tip element fixed to the first tube and the sheath tube, can be effected.

It is also preferred if the switching element, as a fourth step, is operable such that movement of neither the tip element fixed to the first tube nor the sheath tube can be effected.

According to a preferred embodiment, the deployment system of the invention or rather the actuating mechanism comprises a switching element by means of which all four possible step alternatives are provided, preferably in a subsequent row of step 1, following step 2, following step 3, following step 4, with the possibility to switch back an forth.

In this connection it is preferred, if the switching element is a rotary switch or a pressure operable switch.

This measure has the advantage that an easily operatable switching mechanism is provided, which can either be turned or pressed, thus effecting the respective step as a consequence of which the above mentioned actions can be performed. Preferably the rotary switch comprises a marking to indicate which step is performable.

According to a preferred embodiment of the invention, the switching element is attached to a grip system of the deployment system. With this measure, the operator handling the deployment system can easily control and manage the switching mechanism without necessitating additional security or operating steps.

In a preferred embodiment of the deployment system of the invention, the actuating mechanism further comprises a first hollow cylindrical control element for controlling the movement of the sheath tube, the first hollow cylindrical control element being coaxially arranged with respect to the sheath tube and the first tube.

In this connection, it is particularly preferred if the first cylindrical control element comprises a hollow cylindrical outer housing element and a hollow cylindrical inner first bushing element being coaxially arranged with respect to one another, wherein the cylindrical inner first bushing element is, on its proximal end, coupled with the switching element, and on its distal end with the sheath tube.

Presently, and throughout of the invention, a "bushing element" is meant to represent a sleeve-like bearing that is suited to be inserted into a housing to provide a surface

With this measure, i.e. with the first control element for controlling the sheath tube being directly linked with the switching element a direct control for the actuating mechanism via the switching element is provided: The switching element's position controls the position of the first control element and by turning or pressing the switching element a change of position of the first bushing element is effected which in turn directs and controls the operating of the sheath tube.

According to a preferred embodiment, the hollow cylindrical outer housing element has a curved wall comprising a longitudinal slot substantially parallel to the axis of the deployment system.

In this connection, it is also preferred if the hollow cylindrical inner first bushing element has a curved wall comprising a slot forming a path in the curved wall, the path having portions being substantially parallel to the longitudinal axis of the deployment system and having portions being substantially perpendicular to the longitudinal axis of the deployment system.

Presently, the term "slot" or "groove" shall designate a longitudinal and narrow indentation built into a material (here: the curved wall of the bushing element), for the purpose of allowing another material or part (here: a pin element or tooth) to move within the groove/slot and be guided by it.

Also, the term "substantially" as used herein shall mean that a form or design as largely but not essentially exactly the given feature; for example, a substantially parallel line is largely parallel to a fix point but certain minor derivations are also possible, which might e.g. be due to a measuring inaccurateness .

According to another embodiment of the invention, the hollow cylindrical inner first bushing element has a curved wall comprising a slot or groove forming a path in the curved wall, which path has path portions being skew in respect to the longitudinal axis of the deployment system.

This embodiment has the advantage that a smooth transition from the single steps can be performed, as soon as the first and/or the second step are effected via the switching element, without even necessitating an active switching into the third and fourth step.

In the above mentioned embodiments, it is preferred if the first hollow cylindrical control element for controlling the movement of the sheath tube further comprises a pin element having a first end and a second end and being dimensioned and arranged such, that in an axis perpendicular to the deployment system's axis, the pin element's first end projects outwardly from the outer housing element's slot, and the pin element's second end projects inwardly and is engaging with the slot in the curved wall of the inner first bushing element, wherein the pin element is coupled with the proximal end of the sheath tube.

This pin element has the advantage that it functions as a control and guiding peg following the path of the slots in the first bushing element and thus transmitting this movement into a movement of the sheath tube.

Presently, the expression "pin element" is meant to designate any longitudinal, slender, preferably cylindrical, rod-like element that - with its circumference - fits into the slot or grooves in the curved surfaces of the first bushing element and the housing element, and, thus, serves as a guide ping or peg.

According to a further preferred embodiment, the actuating mechanism further comprises a second control element for controlling the movement of the first tube carrying the tip, the second control element being coupled with the first tube.

This measure has the advantage that also the first tube is controllable by means of the switching element, i.e. via the control element's coupling to the first tube and to the switching element.

According to a preferred embodiment, the second control element comprises a mandrel element having different diameters along its length, which mandrel element is coupled with the first tube. In this connection it is preferred if the switching element of the actuating mechanism comprises slots of different sizes, the slots being dimensioned to fit the different diameters of the mandrel element.

Presently, a "mandrel element" is meant to designate an usually tapered or cylindrical axle, spindle, or arbor suitable to be inserted into a slot of the switching element.

With the different diameters along the length of the mandrel element, the mandrel element can fit into the different diameters of the slot, and remains in the respectively fitting slot portions, thereby transmitting a step or movement of the switching element to the mandrel element's different diameters, which in turns effects movement of the first tube.

According to another embodiment, the second control element comprises a second hollow cylindrical inner bushing element being coaxially arranged with first tube and the sheath tube, wherein the cylindrical second inner second bushing element is coupled with the switching element and - eventually via other elements - to the first tube.

As such, the second control element for the first tube is functioning in the same way as the first control element for the sheath tube.

According to a further embodiment of the invention, the hollow cylindrical inner second bushing element has a curved wall comprising a slot/groove forming a path in the curved wall, the path having portions being substantially parallel to the longitudinal axis of the deployment system and having portions being substantially perpendicular to the longitudinal axis of the deployment system.

In a preferred embodiment, the hollow cylindrical inner second bushing element has a curved wall comprising a slot forming a path in the curved wall, which path has path portions being skew in respect to the longitudinal axis of the deployment system.

As described above, this embodiment has the advantage that a smooth transition from the single steps can be performed, as soon as the first and/or the second step are effected via the switching element, without even necessitating an active switching into the third and fourth step.

As described further above, the pin element is moving in the longitudinal slot - which functions as a forced guide path - of the outer housing and the path of the cylindrical inner first bushing. While moving the pin axially in the longitudinal slot the cylindrical inner first bushing is turning automatically due to the skewed path and the forced guide of the pin in the outer housing. The cylindrical inner first bushing is linked to the cylindrical inner second bushing.

Thus, in this embodiment, first und second inner bushing elements are linked to one another, e.g. via a third bushing element. The pins engaging, respectively, with the first and second bushing elements may be oparable from the outside of the housing e.g. via grips - that may be ring-like hollow cylindrical elements surrounding the circumference of the housing. With the actuating mechanism according to the invention and as described, it is only possible to move one grip (and, as a consequence, one pin and one of the first tube or the sheath tube). Only when the end position of a step or a movement is reached with the one grip actuated, the other grip (and, as a consequence the other pin and the other one of the first tube or sheath tube) is free for movement.

As with the first control element described above, it is also preferred if the second hollow cylindrical control element for controlling the movement of the first tube further comprises a second pin element having a first end and a second end and being dimensioned and arranged such, that in an axis perpendicular to the deployment system's axis, the second spin element's first end projects outwardly from the second outer housing element's slot, and the pin element's second end projects inwardly and is engaging with the slot in the curved wall of the inner second bushing element, wherein the second pin element is coupled with the proximal end of the first tube.

Preferably, the first tube is fixed in a fixing sleeve which can move axially within the cylindrical first bushing element. The fixing sleeve as such is - via a connecting element - connected with a moving element which can be axially moved over the housing. The moving element as such is fixedly connected with the handle carrying an actuator lever; by actuating the actuator lever a stepwise movement of the fixing sleeve - and, as a consequence, of the first tube connected therewith - is accomplished. Thereby, the path of movement of the first tube is defined by the path of the cylindrical inner first bushing element.

Thus, the pin element functions as a guiding peg following the path of the slots/groove in the second bushing element and thus transmitting this movement into a movement of the first tube in the distal direction, thus releasing - in a step-by-step mechanism, the proximal end of the prosthesis.

Other objects and advantages of the present invention will become apparent upon reading the following detailed description and appended claims, and upon reference to the accompanying drawings.

BRIEF DESCRIPTION OF THE DRAWINGS

In the drawings,
- Fig. 1A: shows a schematic perspective view of a first exemplary embodiment of the deployment system according to the invention, not drawn to scale, with special emphasis on the first tube, the sheath tube, and the switching element;
- Fig. 1B: shows a schematic perspective view of the first tube of the embodiment of Fig. 1A;
- Fig. 1C: shows an enlarged view of the switching element of Fig. 1A;
- Fig. 1D: shows a partially exploded view of the embodiment of Fig. 1A;
- Fig. 2A: shows a schematic drawing of another exemplary embodiment of a deployment system according to the invention, not drawn to scale;
- Fig. 2B: shows an enlarged view of the distal end of the embodiment shown in Fig. 2A;
- Fig. 2C: shows an enlarged view of the proximal, handling end of the embodiment shown in Fig. 2A,
- Fig. 2D: shows a sectional view of the enlarged view of the distal end of the embodiment shown in Fig. 2B,
- Fig. 2E: shows a sectional view of the proximal, handling end of the embodiment shown in Fig. 2C, depicting first and second control elements for the axial movement of sheath tube and first tube;
- Fig. 2F: shows an enlarged view of the bushing element, being part of the control element for the movement of either the sheath tube and/or the first tube;
- Fig. 3A: shows an enlarged view of another embodiment of a cylindrical inner first and second bushing element, being parts of the control elements for the sheath tube and the first tube, respectively;

- Fig. 3B: shows a perspective view of an embodiment of the cylindrical second bushing element,
- Fig.4: a schematic diagram depicting the switching steps that may be performed according to one embodiment of the invention.

### DETAILLED DESCRIPTION OF THE DRAWINGS

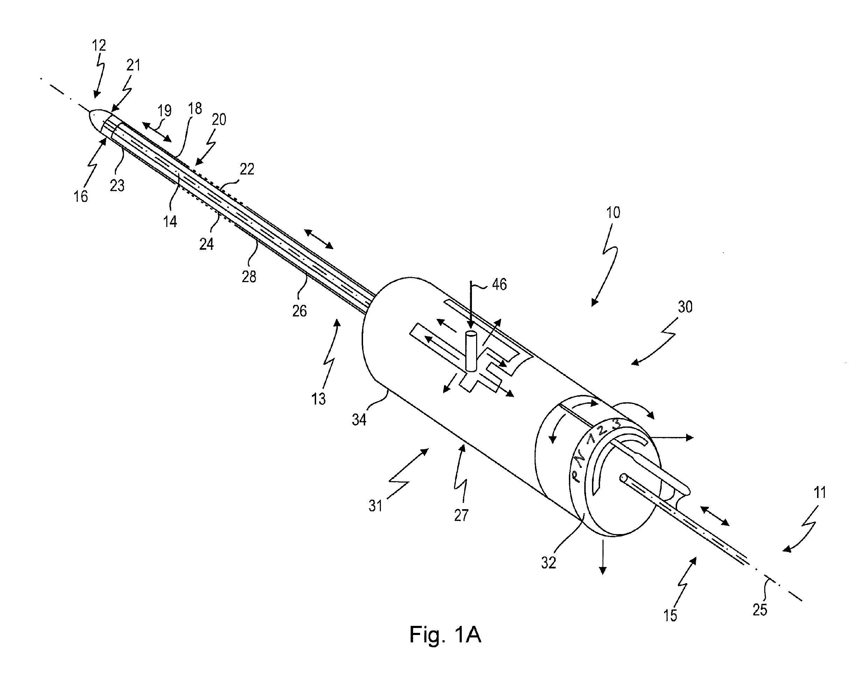

Fig. 1A shows, in a perspective view, a schematic drawing of an exemplary embodiment of a deployment system 10, with a proximal end 11 and a distal end 12 and a longitudinal axis 13, extending from the proximal end 11 to the distal end 12.

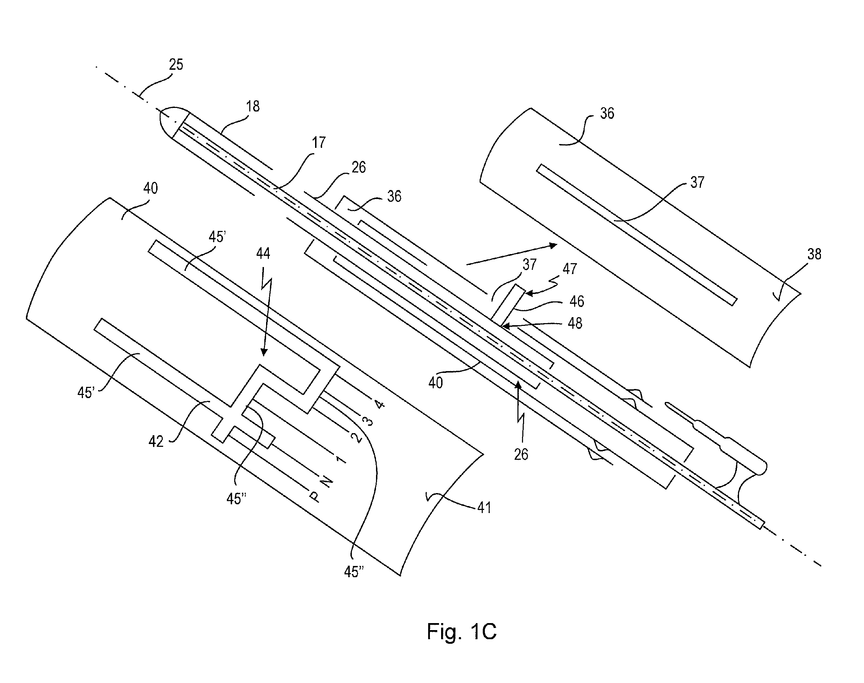

Reference number 14 designates a first tube, having a proximal end 15 and a distal end 16, and a lumen 17 (see fig. 1 C) extending from the proximal end 15 to the distal end 16. Through lumen 17, a guide wire 25 is extending. The distal end 16 fixedly carries a tip or tip element 18, which can be axially moved as indicated by arrow 19. The tip element 18 as such has a proximal end 20 and a distal end 21. The proximal end 20 of the tip element 18 accommodates, in a loaded state, the proximal end 23 of a prosthesis 22, which also has a distal end 24.

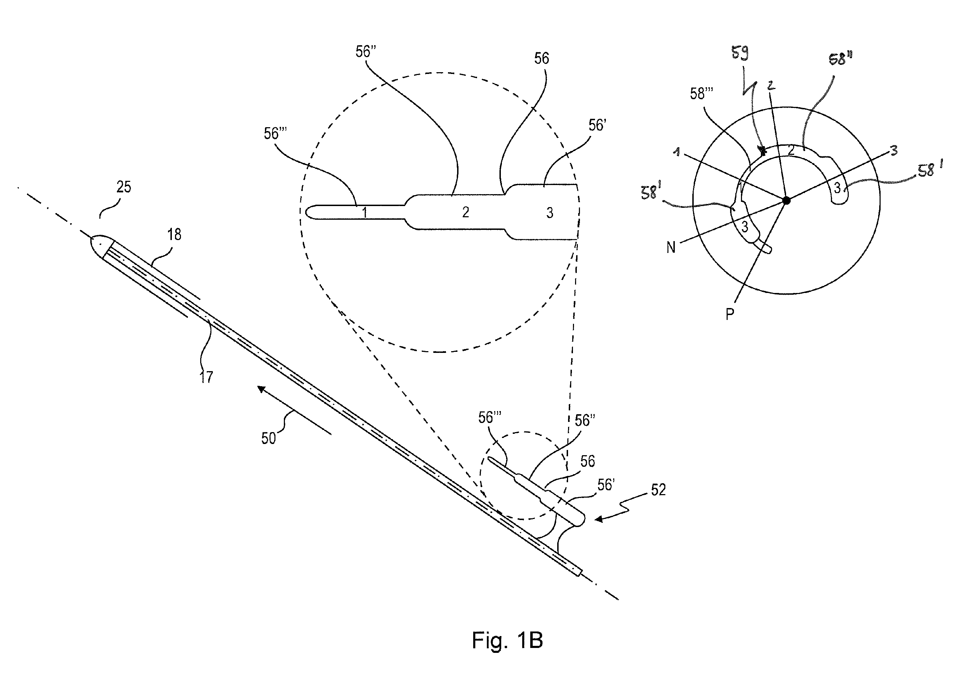

The deployment system also comprises a sheath tube 26 having a proximal end 27 - ending, in the embodiment as shown in Fig. 1, in a handling grip - and a distal end 28, and holding and compressing, with its distal end 28, the distal end 24 of prosthesis 22. In Fig. 1A, the distal end 28 of sheath tube 26 is partially withdrawn (as indicated with the dotted line), thus exposing distal end 24 of prosthesis 22.

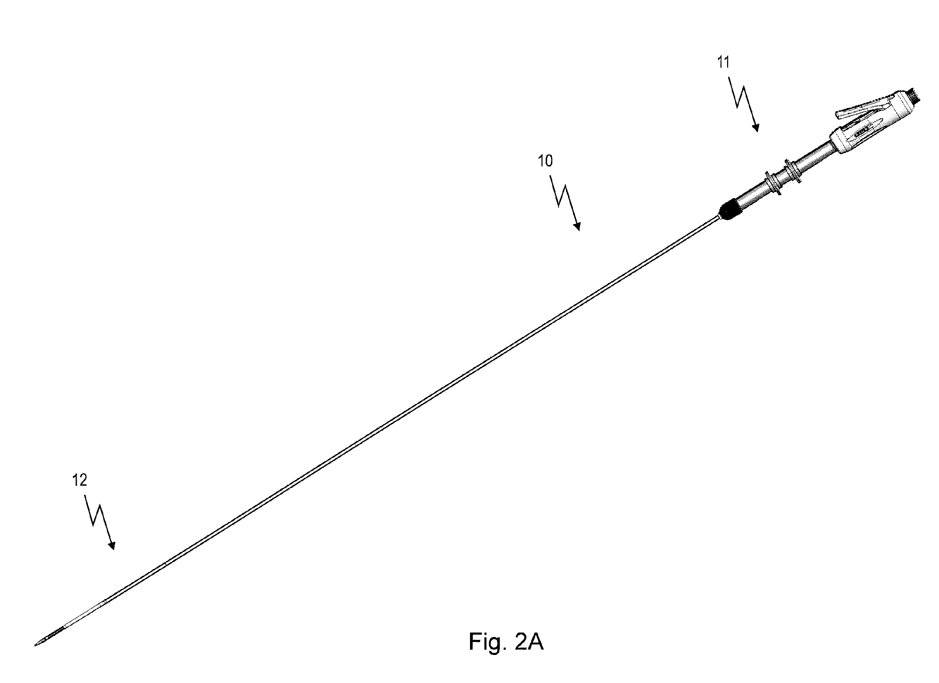

As can be better seen in Fig. 2A, the proximal end 20 of tip element 18 abuts the distal end 28 of sheath tube 26. When the actuating mechanism (see below) is operated, the tip element 18 of the first tube 14 moves in the distal direction, i.e. away from the operator, and the sheath tube 26 can be moved in the proximal direction, i.e. towards the operator, in order to stepwise release and deploy the prosthesis 22 (indicated with the dotted line in Fig. 1A).

The deployment system 10 as depicted in Fig. 1 A further comprises an actuating mechanism 30, comprising a switching element 32, which, in the embodiment of fig. 1, is a rotary switch 32. Switching element 32 (i.e. the rotary switch 32) is generally positioned at the proximal end 11 of deployment system 10, and in particular in the proximal region of handling grip/grip system 31. The switching element 32 can be rotated in positions P, N, 1, 2, 3, 4, as indicated on the switching element 32.

In Fig. 1, reference number 25 designates a guide wire, which is extends through lumen 17.

The actuating mechanism 30 further comprises a first control element 34, which is shown in more detail in Fig. 1C: The first control element 34 comprises a cylindrical outer housing element 36, carrying a slot 37 in its curved wall 38. The first control element 34 is controlling the axial movement of sheath tube 26. The control element 34 further comprises a cylindrical inner first bushing element 40, carrying on its curved wall 41, a groove 42, forming a path 44. As can be seen in Fig. 1C, the path 44 has portions 45' being substantially parallel to the longitudinal axis of the bushing element 40 (and of the deployment system 10), and portions 45" being substantially perpendicular to the longitudinal axis of the bushing element 40 (and of the deployment system 10).

The path 44 has points P, N, 1, 2, 3, 4, corresponding to the respective positions of the switching element 32.

The first control element 34 further comprises a first pin element 46, which is coupled with the proximal end 27 of sheath tube 26, and which protrudes or projects with its first end 47 through the slot 37 of outer housing element 36, and which projects with its second end 48 into the groove 42 of inner bushing element 40. The first pin element 46 follows the path 44 of slot 42, thus controlling the withdrawal steps of sheath tube 26 and, as a consequence of the releasing of prosthesis 22. The inner bushing element 40 is coupled with the switching element 32, which controls the movement of the inner bushing element 40 and directs and controls the path 44 to be followed by the pin element 46.

The pin element 46 is moving in the longitudinal slot 37 - which, thus, functions as a forced guide - of the outer housing element 36 and the skewed path 44 of the cylindrical inner first bushing element 40. While moving the pin element 46 axially in the longitudinal slot 37, the cylindrical inner first bushing element 40 is turning automatically which is due to the skewed path 44 and the forced guide of the pin element 46 in the outer housing 36.

The pin element 46 can, in a preferred embodiment, be dimensioned and designed such, that before it can move along the slot 37 (and along groove 42), it has to be pushed down, i.e. inwardly or towards the center of the deployment system 10, in order to overcome the blocking mechanism. For this purpose, the pin element 46 can have different diameters along its length, with a smaller diameter at its first end 47 and a larger diameter at its second end 48. The blocking mechanism is such that only the smaller diameter fits through and along slot 37, which in turn has a diameter about the size of the smaller diameter of the pin element 46. In that way, the pin element 46 can not be moved if it engages the slot 37 with its larger diameter (not shown).

Fig. 1 B depicts the control mechanism for first tube 14, which, in order to release the proximal end 23 of prosthesis 22 (not depicted in fig. 1 B for sake of clarity), is moved in the distal direction, which is designated with arrow 50 in Fig. 1 B. As can be seen in the embodiment shown in fig. 1 B the deployment system 10 further comprises a second control mechanism 52, comprising - in the embodiment shown here - a mandrel element 56, which has portions 56', 56", and 56'" with different diameters (see enlarged detail in fig. 1 B lower left corner). The portions 56', 56" and 56'" are adapted to engage and fit into portions 58', 58" and 58"', respectively, of a path 58 of slot 59 in switching element 32 (see enlarged detail in fig. 1 B upper right corner).

Thus, if switching element 32 is turned, the rotary movement of the switching element 32 translates into the engagement of the mandrels different portions 56', 56" and 56'" into the different sized slot portions 58', 58" and 58"', thus pushing, in a stepwise manner, first tube 14 and tip element 18 fixedly connected therewith into distal direction, in order to release the proximal end 23 of prosthesis 22 in a last step.

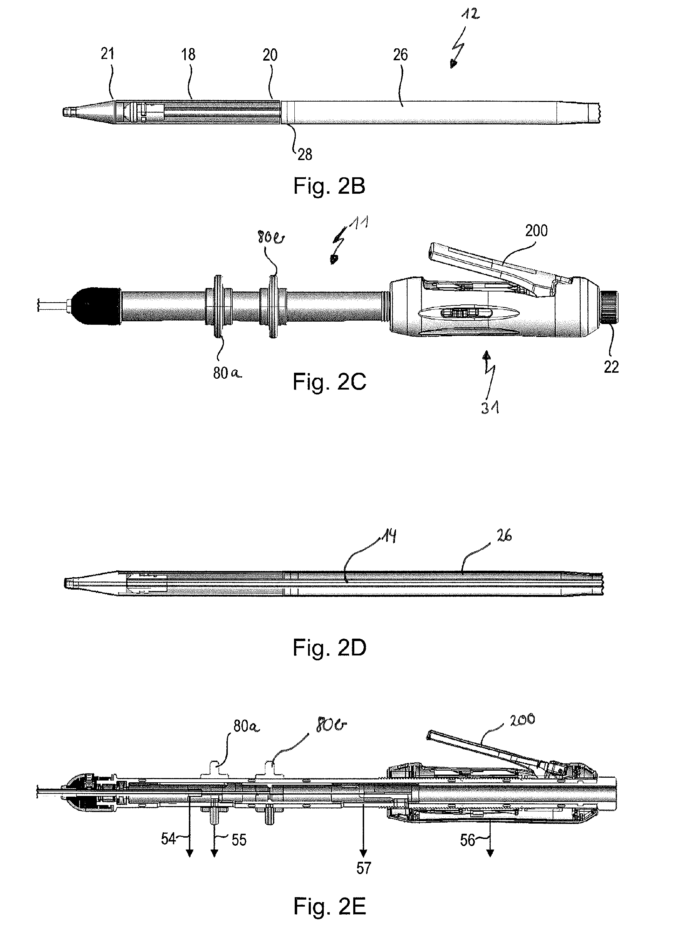

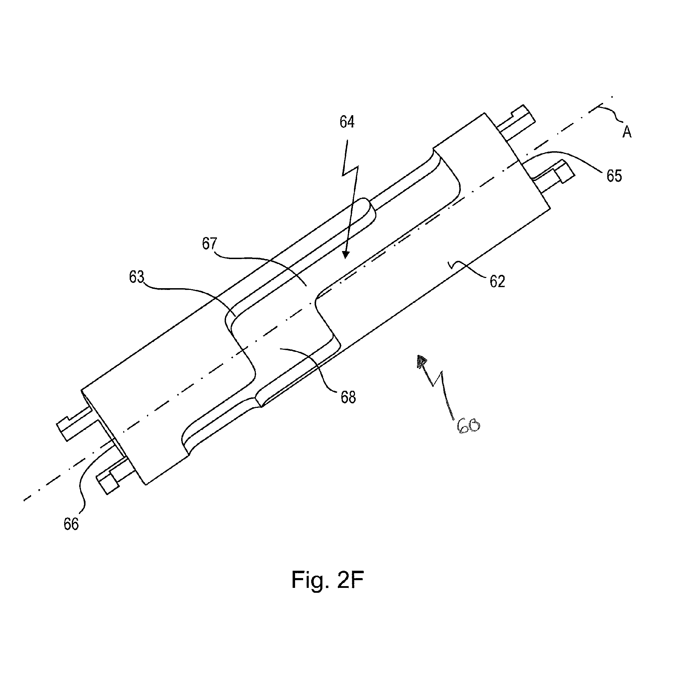

Pin element 46 can be embraced by or be engaged with an operating elements 80a (shown in fig. 2C and 2F) functioning as actuator for withdrawing the sheath tube 26. In the embodiment shown in Fig. 2C, the operating element is an operating ring circumferentially surrounding the outer circumference of the deployment system 10 above the pin element 46 projecting outwardly from the slot 37. The operator can easily grasp the operating element 80a and move it over the slot 37 in order to withdraw the sheath tube 26 from prosthesis 22 in case the axial movement of sheath tube 26 is allowed by the actuating mechanism 30 and switching element 32.

Fig. 2 shows another embodiment of an actuating mechanism 30, wherein in fig. 2 the same elements as in fig. 1 are designated with the same reference numbers.

Fig. 2A shows a side view of the deployment system 10, about drawn to scale, comprising proximal end 11 and distal end 12, as well as longitudinal axis 13 between the ends 11 and 12.

In Fig. 2B, an enlarged view of the distal end 12 of deployment system 10 is depicted, showing tip 18 with its distal end 21 and its proximal end 20. The prosthesis 22 is - for the sake of clarity - not depicted in fig. 2B. As can be seen from fig. 2B, the proximal end 20 of tip 18 abuts distal end 28 of sheath tube 26. Distal end 28 of sheath tube 26 and proximal end 20 of tip element 18 cover and hold and compress prosthesis 22 (not shown) in the loaded state.

Fig. 2C shows an enlarged view of the proximal end 11 of deployment system 10 comprising grip system 31, which, in the embodiment shown in fig. 2 additionally comprises an actuating lever 200, which in the embodiment shown here is coupled with the first tube 14 and which upon actuating effects movement of tip element 18 in the distal direction. Fig. 2C also shows two grip elements (or operator elements) 80a and 80b, which, respectively, embrace a pin element (not shown) and are thus engaged with the pin elements and function as actuator for withdrawing the sheath tube 26 and first tube 14. In the embodiment shown in Fig. 2C, the operating elements 80a, 80b - or grips - are operating rings circumferentially surrounding the outer circumference of the deployment system 10 above the pin elements projecting outwardly. The grips or operating elements 80a, 80b are linked to one another via an internal bushing. The cylindrical inner first bushing element 40 is linked to the cylindrical inner second bushing element 60. In position "D" (i.e. 1, 2, 3 or 4), it is just possible to move one operating element 80a or 80b. Only when the end position with one operating element 80a or 80b is reached, the other operating element 80a, 80b is free for movement.

The enlarged view depicted in Fig. 2D shows a sectional view, giving details about the first tube's 14 extension through deployment system 10, and through sheath tube 26.

Fig. 2E shows an overview for the first and second control elements 34 and 52, controlling the axial movement of sheath tube 26 and first tube 14, respectively: Arrows 54 and 55 designate the control element 34 for the sheath tube, and arrows 56 and 57 designate the control element 52 for the first tube 14.

In fig. 2F, an enlarged view of a part of control mechanism 52 for axial movement of the first tube 14 is depicted, i.e. an inner bushing element 60. Bushing element 60 has a curved wall 62, with a groove 63 forming a path 64 along the circumference of the curved wall 62 of bushing element 60. The bushing element 60 further comprises a proximal end 65 and a distal end 66.

Bushing element 60 functions as part of the control element 52 for the axial movement of the first tube 14 (and of tip element 18). Its proximal end 65 is coupled with the switching element 32, by means of which the operator can operate the switching steps, e.g. by rotating a rotary switch from a first position (e.g. "P") to a second position (e.g. "N" or "1"). This rotary movement translates in a rotary movement of bushing element 60 which engages via its proximal end 65 with the switching element 32. A pin element or tooth (not shown in fig. 2F) engaging with the groove 63 on the curved wall 62 of bushing element 60 is being guided in the path 64 of the groove, once the bushing element is rotated about its longitudinal axis A. The movement of the pin element then translates either in the axial movement of the first tube 14, which can be effected by actuating the lever 200, which, in the embodiment shown in Fig. 2, is coupled with the bushing 60.

As can be seen from fig. 2F, the path 64 the groove 63 has portions 67 being substantially parallel to the longitudinal axis of the bushing element 60 (and of the deployment system 10), and portions 68 being substantially perpendicular to the longitudinal axis of the bushing element 60 (and of the deployment system 10).

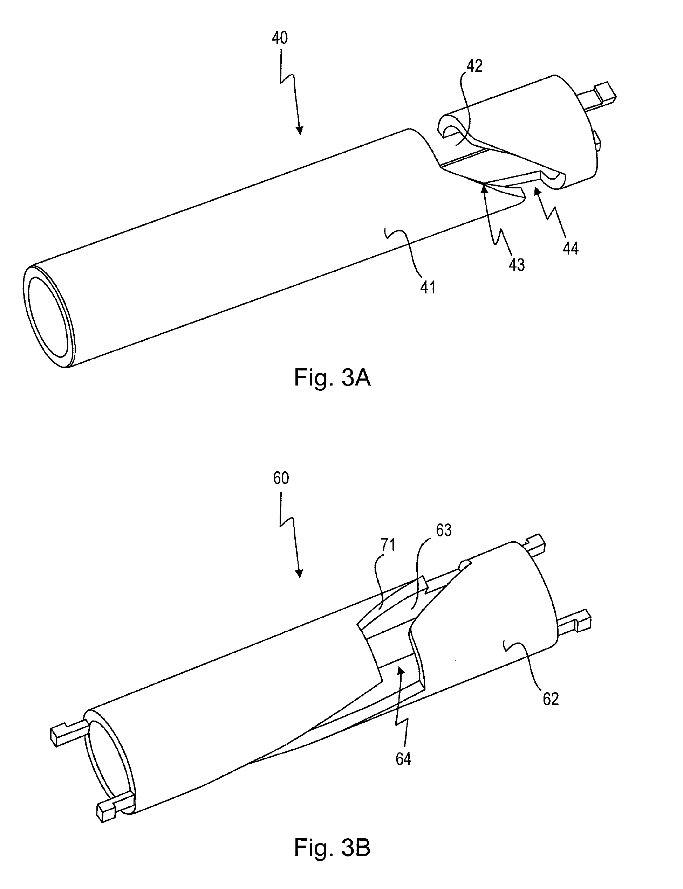

Fig. 3A and 3B show details of other embodiments of bushing elements 40, 60: Fig. 3A shows another embodiment of the first inner bushing element 40 which is part of the control element 34 for the axial movement of the sheath tube 26. The bushing element 40 has a slot or groove 42 formed in the curved wall 41 of the bushing element 40. The slot/groove 42 forms a path 44 on the curved wall, wherein the path, in the embodiment shown in fig. 3A, is skew (indicated with 43 in Fig. 3A) with respect to the longitudinal axis of the bushing element 40 and of the deployment system 10.

Fig. 3B depicts another embodiment of the second inner bushing element 60 which is part of the control element 52 for the axial movement of the first tube 14 (and, as a consequence of tip element 18). The bushing element 60 has a slot or groove 63 formed in the curved wall 62 of the bushing element 60. The slot/groove 63 forms a path 64 on the curved wall, wherein the path, in the embodiment shown in fig. 3A, is skew (indicated with reference number 71) with respect to the longitudinal axis of the bushing element 60 and of the deployment system 10.

The skew path of the bushing elements 40 and 60 has the advantage that a smooth transition for the single deployment steps is provided, so that a pin or tooth engaging with the groove/slot can move along the path without necessitating a separate actuating for each movement.

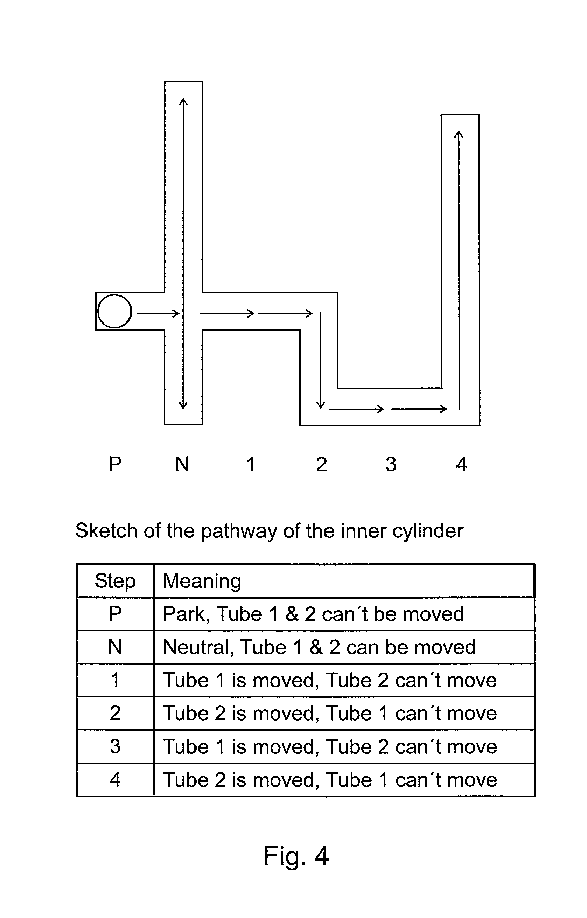

Fig. 4 shows a diagram depicting the switching steps that can be performed with an exemplary embodiment of the deployment system according to the invention: In the upper picture, an exemplary path of travel is depicted, wherein it is to be understood, that the path is merely exemplary and a person skilled in the art will, from the teaching presented herein, readily find other paths or switching steps suitable for the present invention. In particular, the path can comprise portions that are parallel and perpendicular to the longitudinal axis of the bushing element/deployment system, and/or portions that are skew with respect to the longitudinal axis of the bushing elements/deployment system.

In fig. 4, the single switching steps or positions are designated with "P", "N"; "1 ", "2", "3", and "4", each position effecting different deployment steps and separately controlling the axial movement of the first tube comprising the tip and of the sheath tube:

In position/switching step "P" neither the first tube 14 nor the sheath tube 26 can be moved, and this position is chosen, e.g. when the deployment system is to be shipped. Position "N" is chosen, when both, the first tube 14 and the sheath tube 26 are to be moved, e.g. for loading a prosthesis onto the deployment system.

In position "1", the first tube 14 can be moved, while movement of the sheath tube 26 is blocked/prevented. Thus, the tip element 18 can move in the distal direction. In position "2", the sheath tube 26 can be moved, while first tube 14 cannot move, thus effecting a withdrawal of sheath tube 26 from the distal end of prosthesis 22.

In step/position 3, the first tube 14 can be moved, while sheath tube 26, again, is blocked from moving, thus moving the tip again further in distal direction, releasing the proximal end of prosthesis 22. In a last position 4, the sheath tube 26 can be moved, and the first tube 14 is blocked, so that the sheath tube can be completely withdrawn from prosthesis 22 thus releasing it.

For actuating the deployment system 10, the following steps are being made: The operator actuates the switching element 32, which is, in the embodiment shown in Fig. 1, proximal fixed onto grip system 31. The switching element 32 carries markers for the respective positions P, N, 1, 2, 3 and 4. Whereas the movement strategy is, in principal, free to define, in the figures presented herein, the release mechanism for an implant is described. The markings P, N, 1, 2, 3, and 4 are allocated to a path on the curved wall of an inner cylinder, i.e. bushing 40, 60. When actuating the switching element 32, i.e. when rotating the rotary switch, the pattern of the path of the bushing element 40, 60 is moved simultaneously, since the inner cylinder/bushing element 40, 60 are coupled with the switching element 32. Thus, the movement is defined via the inner bushing's slot 42 path 44.

The movement of sheath tube 26 is, e.g., performed and effected by moving pin element 46 (indicated in Fig. 4 as a filled circle in position "P"). As described above the pin element 46 can designed such that it engages with an operating element, e.g. ring 80, for ease of handling. The pin element 46 is coupled with sheath tube 26; as can be seen from fig. 4, the pin element 46 can be moved axially when the path is in position/step "N", "2", and "4". The maximal movement of sheath tube 26 is limited by the path given on the inner bushing element 40.

The movement of the first tube 14 is performed and effected by moving, e.g. mandrel element 56 (see Fig. 1) being coupled with first tube 14 and situated at the proximal end of the grip system 31. It is possible to move the mandrel when the path is in position/steps 1 and 3. The maximum movement of first tube 14 is limited by the mandrels element 56 different diameters 56', 56", and 56'" which have to pass different slot sizes 58', 58" and 58"' integrated in the switching element 32, here the rotary switch.

In the alternative embodiment, where the movement of the first tube 14 is effected by a second inner bushing, the movement of the first tube 14 will be effected as described above for movement of sheath tube 26. A pin element or tooth 46 of the deployment system 10 is moved in the longitudinal slot 42 - which functions as a forced guide - of the outer housing 36 and the (a) skewed path 44 or (b) straight path 64 of the cylindrical inner first bushing 40. (a): When moving the pin element 46 axially in the longitudinal slot 37, the cylindrical inner first bushing 40 is turning automatically which is due to the skewed path 44 and the forced guide of the pin element 46 in the outer housing 36. The cylindrical inner first bushing 40 is linked to the cylindrical inner second bushing 60. In position "D" (i.e. 1, 2, 3 or 4) it is just possible to move one, i.e. the switching element 32 or the control element. Only when the end position with one, the first 34 or the second 52 control element is reached the respectively other control element is free for movement. (b): In the embodiment with the straight path, the pin element 46 can be moved until the end position of the first step is reached; only then it is possible to turn or rotate the switching element 32 to the next position to go ahead with the next step in the deployment procedure.

While the invention is suitable for the deployment of a cardiac valve prosthesis, a person skilled in the art will, when reading the present disclosure, readily recognize that the deployment system according to the invention may also be applied for any other prosthesis, too.

## Claims

1. Deployment system (10) for deploying a self expandable prosthesis (22), the deployment system (10) having a proximal (11) and a distal (12) end, and a longitudinal axis (13) extending from the proximal (11) to the distal (12) end, and further comprising:
a first tube (14) comprising a lumen (17), and comprising a tip element (18) having a proximal (20) and a distal (21) tip element end, the tip element (18) being fixedly connected to the first tube (14) at a distal end (16) of the first tube (14), wherein the tip element (18) is designed such, that a proximal end (23) of a prosthesis (22) is detachably accomodatable and holdable by and within the tip element (18), wherein the tip element (18) is slidable distally relative to the prosthesis (22),
a sheath tube (26) tube having a proximal (27) and a distal (28) sheath tube end, the sheath tube (26) being designed such, that a distal end (24) of the prosthesis (22) is detachably holdable and compressable by and within the distal sheath tube end (28), wherein the sheath tube (26) is slidable proximally relative to the prosthesis (22), to release the distal end (24) of the prosthesis (22)
wherein the first tube (14) and the sheath tube (26) are coaxially arranged with respect to one another and are axially moveable with respect to one another, and wherein the tip element (18) and the sheath tube (26), in a loaded state of the prosthesis (22), are arranged such, that the proximal tip element end (20) and the distal sheath tube end (28) abut to one another or that the proximal tip element end (20) is slidable over the distal sheath tube end (28), to release and expand the prosthesis (22),
**characterized in that** the deployment system (10) further comprises
an actuating mechanism (30) by means of which the axial movement of the tip element (18) releasing the proximal end (23) of the prosthesis (22) and the axial movement of the sheath tube (26) releasing the distal end (24) of the prosthesis (22) are operated and controlled separately, wherein the actuating mechanism (30) comprises a switching element (32) being operable to perform different switching steps (P, N, 1, 2, 3, 4).

2. The deployment system (10) of claim 1, **characterized in that** the switching element (32), as one switching step, is operable such that the movement of the tip element fixed to the first tube is effected when the movement of the sheath tube is blocked.

3. The deployment system (10) of claim 1 or 2, **characterized in that** the switching element (32), as a second switching step, is operable such that the movement of the tip element (18) fixed to the first tube (14) is blocked when the movement of the sheath tube (26) is effected.

4. The deployment system (10) of any of claims 1 or 3, **characterized in that** the switching element (32), as a third switching step, is operable such that movement of both, the tip element (18) fixed to the first tube (14) and the sheath tube (26), can be effected.

5. The deployment system (10) of any of claims 1 to 4, **characterized in that** the switching element (32), as a fourth step, is operable such that movement of neither the tip element (18) fixed to the first tube (14) nor the sheath tube (26) can be effected.

6. The deployment system (10) of any of claims 1 to 5, **characterized in that** the switching element (32) is a rotary switch or a pressure operable switch.

7. The deployment system (10) of any of claims 2 to 6, **characterized in that** the switching element (32) is attached to a grip system (31) of the deployment system (10).

8. The deployment system (10) of any of claims 1 to 7, **characterized in that** the actuating mechanism (30) further comprises a first hollow cylindrical control element (34) for controlling the movement of the sheath tube (26), the first hollow cylindrical control element (34) being coaxially arranged with respect to the sheath tube (26) and the first tube (14).

9. The deployment system (10) of claim 8, **characterized in that** the first cylindrical control element (34) comprises a first hollow cylindrical outer housing element (36) and a hollow cylindrical inner first bushing element (40) being coaxially arranged with respect to one another, wherein the cylindrical inner first bushing element (40) is coupled with the switching element (32).

10. The deployment system (10) of claim 9, **characterized in that** the first hollow cylindrical outer housing element (36) has a wall (38) comprising a longitudinal slot (37) substantially parallel to the axis (13) of the deployment system (10).

11. The deployment system (10) of claim 9 or 10, **characterized in that** the hollow cylindrical inner first bushing element (40) has a wall (41) comprising a groove (42) forming a path (44) in the curved wall (41), the path (44) having portions (45') being substantially parallel to the longitudinal axis (13) of the deployment system (10) and having portions (45") being substantially perpendicular to the longitudinal axis (13) of the deployment system (10).

12. The deployment system (10) of claim 9 or 10, **characterized in that** the hollow cylindrical inner first bushing element (40) has a wall (41) comprising a groove (42) forming a path (44) in the curved wall (41), which path (41) has path portions (43) being skew in respect to the longitudinal axis (13) of the deployment system (10).

13. The deployment system (10) of any of claims 8 to 12, **characterized in that** the first hollow cylindrical control element (34) for controlling the movement of the sheath tube (26) further comprises a first pin element (46) having a first end (47) and a second end (48) and being dimensioned and arranged such, that in an axis perpendicular to the deployment system's longitudinal axis (13), the first pin element's first end (47) projects outwardly from the first outer housing element's slot (37), and the first pin element's second end (48) projects inwardly and is engaging with the groove (42) in the curved wall (41) of the inner first bushing element (40), wherein the first pin element (46) is coupled with the proximal end (27) of the sheath tube (26).

14. The deployment system (10) of any of claims 1 to 13, **characterized in that** the actuating mechanism (30) further comprises a second control element (52) for controlling the movement of the first tube (14) carrying the tip (18), the second control element (52) being coupled with the first tube (14).

15. The deployment system (10) of claim 14, **characterized in that** the second control element (52) comprises a mandrel element (56) having different diameters (56', 56", 56"') along its length, which mandrel element (56) is coupled with the first tube (14), and that the switching element (32) comprises slots of different sizes (58', 58", 58"'), the slots (58', 58", 58"')being dimensioned to fit the different diameters (56', 56", 56"')of the mandrel (56).

16. The deployment system (10) of claim 14, **characterized in that** the second control element (52) comprises a hollow cylindrical second inner bushing element (60) being coaxially arranged with respect to the first tube (14) and the sheath tube (26), wherein the cylindrical second inner bushing element (60) is coupled with the switching element (32) and the first tube (14).

17. The deployment system (10) of claim 16, **characterized in that** the hollow cylindrical inner second bushing element (60) has a wall (62) comprising a groove (63) forming a path (64) in the curved wall (62), the path (64) having portions (67) being substantially parallel to the longitudinal axis (13) of the deployment system (10) and having portions (68) being substantially perpendicular to the longitudinal axis (13) of the deployment system (10).

18. The deployment system (10) of claim 16, **characterized in that** the hollow cylindrical inner second bushing element (60) has a wall (62) comprising a groove (63) forming a path (64) in the curved wall (62), which path (64) has path portions (71) being skew in respect to the longitudinal axis (13) of the deployment system (10).

19. The deployment system (10) of any of claims 16 to 18, **characterized in that** the second hollow cylindrical control element for controlling the movement of the first tube (14) further comprises a second pin element having a first end and a second end and being dimensioned and arranged such, that in an axis perpendicular to the deployment system's axis, the second pin element's first end projects outwardly from the second outer housing element's slot, and that the pin element's second end projects inwardly and is engaging with the groove in the curved wall of the inner second bushing element (60), wherein the second pin element is coupled with the proximal end of the first tube (14).

20. The deployment system (10) of claim 20, **characterized in that** the first (34) and the second (52) hollow cylindrical control element are linked to one another via a third bushing element.

21. The deployment system (10) of any of claims 16 to 20, **characterized in that** the first (34) and the second (52) hollow cylindrical control element are linked with operable elements (80a, 80b) provided on the outer circumference of the deployment system (10).
